# EUROPEAN PATENT SPECIFICATION

(11) **EP 4 090 310 B1**
(45) Date of publication and mention of the grant of the patent: **10.05.2023**
(21) Application number: 21701238.4
(22) Date of filing: 12.01.2021
(51) Int. Cl.: A61K 8/44, A61K 8/46, A61Q 5/00

(54) **HAIR TREATMENT COMPOSITION**
HAARBEHANDLUNGSZUSAMMENSETZUNG ENTHALTEND EINE PIROCTON-VERBINDUNG
COMPOSITION DE TRAITEMENT CAPILLAIRE COMPRENANT UN COMPOSÉ PIROCTONE

(30) Priority: 17.01.2020 EP 20152350
(43) Date of publication of application: 23.11.2022
(73) Proprietor: Unilever IP Holdings B.V., 3013 AL Rotterdam (NL); Unilever Global IP Limited, Wirral, Merseyside CH62 4ZD (GB)
(72) Inventor: AINGER., Nicholas, John, Wirral Merseyside CH63 3JW (GB); COLLINS, Luisa, Zoe, Wirral Merseyside CH63 3JW (GB); DAWSON, Joanna, Susan, Wirral Merseyside CH63 3JW (GB); FORREST, Richard, Aaron, Wirral Merseyside CH63 3JW (GB); ROBERTS, Louise, Jannette, Wirral Merseyside CH63 3JW (GB); WHITEHEAD, Paul, Stephen, Wirral Merseyside CH63 3JW (GB)
(74) Representative: Tansley, Sally Elizabeth
(86) International application number: PCT/EP2021/050497
(87) International publication number: WO 2021/144272

(56) References cited:
- DE-A1-102010 055 763
- DE-A1-102012 220 148
- JP-A- 2001 254 095
- JP-A- 2005 232 113
- US-A1- 2017 165 169
- US-A1- 2019 000 735
- US-A1- 2019 105 244
- US-A1- 2020 000 690

## Description

### Field of the Invention

The present invention relates to a hair treatment composition, particularly an anti-dandruff shampoo composition.

### Background of the Invention

Dandruff is a problem affecting many globally. The condition is manifested by the shedding of clumps of dead skin cells from the scalp, these are white in colour and provide an aesthetically displeasing appearance. A factor that contributes to dandruff are certain members of the *Malassezia* yeasts. To combat these yeasts, hair treatment compositions are developed including various actives for their antidandruff effectiveness. Piroctone compound such as piroctone olamine is one such active.

A common problem with piroctone compound is that deposition of the active onto the desired surface during wash process is minimum. The desired surface is typically scalp and/or hair. For example, piroctone compound such as piroctone olamine is usually soluble in surfactants of the cleansing phase comprised in a hair treatment composition. During the excessive rinsing process, the majority of piroctone is likely to be washed away together with the surfactants. Poor deposition is correlated with low antidandruff activity, thus little mitigation of the ill-effects of dandruff. To date, there are attempts to offset this drawback by increasing the level of piroctone olamine in hair treatment composition. Such approach causes a variety of issues such as increased costs, potential instability of the formulation and potential adverse effect to hair sensory. Hence it is not an approach favoured by the industry.

DE102012203240A1 describes hair treatment composition with increased antidandruff effect and improved combing, gloss, elasticity of hair. The composition includes antidandruff agent selected from zinc pyrithione, climbazole, octopirox, ketoconazol, selenium disulphide, selenium containing vegetable oil, selenium containing plant extract, and a cationic amino silicone. Examples include shampoos and conditioners comprising Octopirox^{®}(Piroctone Olamine), amino silicone and surfactants.

US2013/0059929A1 describes a cosmetic or dermatological preparation, wherein the preparation comprises at least one of benzethonium chloride, methylisothiazolinone, piroctone olamine, and lauroyl ethyl arginate, and does not contain a preservative that comprises a phenolic group. The preparations show improved sensory properties and sufficient microbiological stability. US2002/0035161A1 describes a cosmetic/pharmaceutical oil-in-water emulsion including a discontinuous fatty phase dispersed in a continuous aqueous phase and comprising an effective amount of at least one biologically active agent (A) and an effect amount of an emulsifying system (B) therefore, said at least one biologically active (A) being non-solubilized therein in micronized particulate state, at least 80%, numerically, of said micronized particles having diameters ranging from 1 to 10 µm and at least 50%, also numerically, having diameters of less than 5 µm.

US 2020/000690 discloses foam compositions with low levels of surfactants and surfactant solulble anti-dandruff compounds. US 2019/000735 A1 (KELLY CASEY PATRICK [US] ET AL) 3 January 2019 discloses a piroctone shampoo.

Despite all the prior art, there remains a need to improve the deposition of piroctone compounds, especially piroctone acid or piroctone olamine, onto the surface of scalp and/or hair during washing process. There also remains a further need to improve the deposition onto said surface without adverse side effects to sensory, formulation rheology and conditioning performance.

### Description of the Invention

The present invention relates to a personal cleansing composition: according to Claim 1.

The invention also relates to a non-therapeutic method of treating hair or the scalp, comprising application of the personal care composition described above.

The invention further relates to the therapeutic use of the composition described above to enhance the deposition of a piroctone compound.

For the avoidance of doubt, any feature of one aspect of the present invention may be utilised in any other aspect of the invention. Any feature described as 'preferred' should be understood to be particularly preferred in combination with a further preferred feature or features. Herein, any feature of a particular embodiment may be utilised in any other embodiment of the invention. The word 'comprising' is intended to mean 'including' but not necessarily 'consisting of' or 'composed of'. In other words, the listed steps or options need not be exhaustive. The examples given in the description below are intended to clarify the invention but not to limit the invention. All percentages are weight/weight percentages unless otherwise indicated. Except in the operating and comparative examples, or where otherwise explicitly indicated, all numbers in this description indicating amounts of material or conditions of reaction, physical properties or materials and/or use are to be understood as modified by the word 'about'.

### Detailed Description of the Invention

The piroctone compound for use in the present invention include piroctone acid, primary, secondary and tertiary olamine salts of piroctone acid (such as the diethanolamine and triethanolamine salts), and mixtures thereof, preferably piroctone acid, primary olamine salt of piroctone acid (i.e. piroctone olamine, also known as Octopirox^{®}) and mixtures thereof.

Piroctone Olamine is an olamine salt of the hydroxamic acid derivative piroctone. It is commonly known as piroctone ethanolamine with the trade name Octopirox^{®}.

The piroctone olamine according to the present invention is a 1:1 compound of 1-hydroxy-4-methyl-6-(2,4,4-trimethylpentyl)-2(1*H*)-pyridinone with 2-aminoethanol and is also designated 1-hydroxy-4-methyl-6-(2,4,4-trimethylpentyl)-2(1*H*) pyridinone monoethanolamine salt. The CAS number is 68890-66-4 and the compound has the general formula (I) as below:

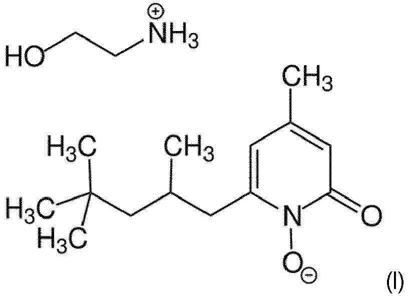

The piroctone compound, particularly the piroctone olamine is preferably present from 0.01 to 5% by weight of the total composition, more preferably from 0.1 to 5 wt%, most preferably from 0.2 to 3 wt%, of the total composition.

The cosmetic composition comprises one cleansing surfactants. Surfactants are compounds which have hydrophilic and hydrophobic portions that act to reduce the surface tension of the aqueous solutions they are dissolved in. The cleansing surfactant comprises an amphoteric surfactant and an ethoxylated anionic surfactant in which the anionic surfactant has an average degree of ethoxylation of 3 or greater

The alkyl chain of the ethoxylated anionic surfactant is preferably from 8 to 18, more preferably from 10 to 16 carbon atoms and may be unsaturated. Preferred alkyl ether sulphates are those of formula (I):

R-O-(CH₂CH₂-O)ₙ-SO₃⁻M⁺ (I)

wherein R is a straight or branched alkyl chain having 8 to 18 (preferably 12 to 18) carbon atoms; n is the average degree of ethoxylation is 3 or greater, preferably 3 (and M is a solubilizing cation such as sodium, potassium, ammonium or substituted ammonium. A preferred example is sodium lauryl ether sulphate (SLES). The most preferred example is SLES having an average degree of ethoxylation of 3 or greater, preferably an average degree of ethoxylation of 3, preferably the surfactant is sodium laureth sulphate (3EO).

Preferred amphoteric or zwitterionic cleansing surfactants including; alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Preferred amphoteric surfactants include, cocodimethyl sulphopropyl betaine, lauryl betaine, coco betaine, cocamidopropyl betaine and sodium cocoamphoacetate. A particularly preferred is amphoteric surfactant is cocamidopropyl betaine.
the ratio of ethoxylated anionic surfactant to amphoteric surfactant is from 3:1 to 4:1.

The total amount of cleansing surfactant in a shampoo composition for use in the invention is from 6 to 12% by total weight surfactant based on the total weight of the composition.

Non-limiting examples of further cleansing anionic surfactants, which may be present but are not preferred are alkyl sulphates, alkaryl sulphonates, N-alkyl sarcosinates, alkyl phosphates, alkyl ether phosphates, acyl amino acid based surfactants, alkyl ether carboxylic acids, acyl taurates, acyl glutamates, alkyl glycinates and salts thereof, especially their sodium, magnesium, ammonium and mono-, di- and triethanolamine salts. The alkyl and acyl groups in the preceding list generally contain from 8 to 18, preferably from 10 to 16 carbon atoms and may be unsaturated.

Further non-limiting examples of cleansing surfactants may include non-ionic cleansing surfactants including; aliphatic (C₈ - C₁₈) primary or secondary linear or branched chain alcohols with alkylene oxides, usually ethylene oxide and generally having from 6 to 30 ethylene oxide groups. However, it is preferred if non-ionic cleansing surfactants are not present. Other representative cleansing surfactants include mono- or di-alkyl alkanolamides (examples include coco mono-ethanolamide and coco mono-isopropanolamide) and alkyl polyglycosides (APGs). Suitable alkyl polyglycosides for use in the invention are commercially available and include for example those materials identified as: Plantapon 1200 and Plantapon 2000 ex BASF. Other sugar-derived surfactants, which can be included in compositions for use in the invention include the C₁₀-C₁₈ N-alkyl (C₁-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

Mixtures of any of the foregoing anionic, non-ionic and amphoteric cleansing surfactants may also be suitable, preferably where the primary to secondary surfactant ratio is between 1:1 - 10:1, more preferably 2:1 - 9:1 and most preferably 3:1 - 8:1, based on the inclusion weight of the cleansing surfactant in the shampoo composition.

The personal care composition may comprise at least one cationic deposition polymer. Suitable cationic polymers may be homopolymers which are cationically substituted or may be formed from two or more types of monomers. The weight average (M_{w}) molecular weight of the polymers will generally be between 100 000 and 2 million daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus, when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general, secondary and tertiary amines, especially tertiary, are preferred.

Amine substituted vinyl monomers and amines can be polymerised in the amine form and then converted to ammonium by quaternization.

The cationic polymers can comprise mixtures of monomer units derived from amine- and/or quaternary ammonium-substituted monomer and/or compatible spacer monomers.

Suitable (non-limiting examples of) cationic polymers include:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquatemium 6 and Polyquatemium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides(as described in WO95/22311).

Other cationic polymers that can be used include cationic polysaccharide polymers, such as cationic cellulose derivatives, cationic starch derivatives, and cationic guar gum derivatives.

Cationic polysaccharide polymers suitable for use in compositions for use in the invention include monomers of the formula:

A-O-[R-N⁺(R¹)(R²)(R³)X-],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquatemium 24. These materials are available from the Amerchol Corporation, for instance under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581). Examples of such materials include the polymer LR and JR series from Dow, generally referred to in the industry (CTFA) as Polyquaternium 10.

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14 and JAGUAR C17.

Mixtures of any of the above cationic polymers may be used.

Cationic polymer will generally be present in a shampoo composition for use in the invention at levels of from 0.01 to 5%, preferably from 0.02 to 1%, more preferably from 0.05 to 0.8% by total weight of cationic polymer based on the total weight of the composition.

Preferably the composition of the invention, particularly an aqueous shampoo composition for use in the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent will generally be present in a shampoo composition for use in the invention at levels of from 0.1 to 10%, preferably from 0.15 to 6%, more preferably from 0.2 to 4% by total weight of suspending agent based on the total weight of the composition.

The cosmetic composition may optionally comprise one or more components, provided that the optional components are physically and chemically compatible with the essential components described hereinbefore, and do not otherwise unduly impair sensory, formulation rheology and conditioning performance. Individual concentrations of such optional components may range from 0.001 % to 10% by weight of the total composition, preferably from 0.01% to 5%wt%. Such components may include conditioning agents, fragrance, dyes, pigments, pH adjusting agents, peariescers or opacifiers, viscosity modifiers, preservatives, and natural hair nutrients such as botanicals, fruit extracts, sugar derivatives and amino acids.

One of the preferred optional components is a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich. Suitable cross-linked polymers of acrylic acid and acrylate esters are Pemulen TR1 or Pemulen TR2. A suitable heteropolysaccharide gum is xanthan gum, for example that available as Kelzan mu. Mixtures of any of the above suspending agents may be used. Preferred is a mixture of cross-linked polymer of acrylic acid and crystalline long chain acyl derivative.

Suspending agent, if included, will generally be present in the composition at a level of from 0.01 to 5 wt.%, preferably from 0.1 to 2.5 wt.%, more preferably from 0.25 to 1 wt.%.

Another preferred optional component is a conditioning agent, providing conditioning benefit. Typically, the most popular conditioning agents used in cosmetic compositions are water-insoluble oily materials such as mineral oils, naturally occurring oils such as triglycerides and silicone oils. Conditioning benefit is achieved by the oily material being deposited onto the hair resulting in the formation of a film, which makes the hair more lubricated and less dry. Preferably, the conditioning agent is non-volatile, meaning that it has a vapour pressure of less than 1000 Pa at 25°C.

Preferably, the composition comprises discrete dispersed droplets of a water-insoluble conditioning agent, which has a mean droplet diameter (D_{3,2}) of less than 50 microns, preferably less than 30 microns, more preferably less than15 microns, most preferably less than 10 microns. The mean droplet diameter (D_{3,2}) of a water-insoluble conditioning agent may be measured by means of a laser light scattering technique, for example using a 2600D Particle Sizer from Malvern Instruments. The water-insoluble conditioning agent may include non-silicone conditioning agent comprising non-silicone oily or fatty materials such as hydrocarbon oils, fatty esters and mixtures thereof. Preferably, the water-insoluble conditioning agent is emulsified silicone oil.

Suitable silicones include polydiorganosiloxanes, in particular polydimethylsiloxanes which have the CTFA designation dimethicone. Also suitable for use in compositions of this invention (particularly shampoos and conditioners) are polydimethyl siloxanes having hydroxyl end groups, which have the CTFA designation dimethiconol. Also suitable for use in compositions of this invention are silicone gums having a slight degree of cross-linking, as are described for example in WO 96/31188. Preferably, the silicone oil comprises dimethicone, dimethiconol or a mixture thereof.

The viscosity of the emulsified silicone itself (not the emulsion or the final hair care composition) is typically at least 10,000 cSt (centi-Stokes=mm²·S⁻¹) at 25°C, preferably at least 60,000 cSt, most preferably at least 500,000 cSt, ideally at least 1,000,000 cSt. Preferably the viscosity does not exceed 10⁹ cSt for ease of formulation. Suitable methods for measuring the kinematic viscosity of silicone oils are known to those skilled in the art, e.g. capillary viscometers. For high viscosity silicones, a constant stress rheometer can be used to measure viscosity.

Suitable emulsified silicones for use in the compositions are available as pre-formed silicone emulsions from suppliers of silicones such as Dow Coming and GE silicones. The use of such pre-formed silicone emulsion is preferred for ease of processing and control of silicone particle size. Such pre-formed silicone emulsions will typically additionally comprise a suitable emulsifier and may be prepared by a chemical emulsification process such as emulsion polymerisation, or by mechanical emulsification using a high shear mixer.

Examples of suitable pre-formed silicone emulsions include DC1785, DC1788, DC7128, all available from Dow Corning. These are emulsions of dimethiconol/dimethicone.

Another class of silicones which may be used are functionalized silicones such as amino functional silicones, meaning a silicone containing at least one primary, secondary or tertiary amine group, or a quaternary ammonium group. Examples of suitable amino functional silicones include polysiloxanes having the CTFA designation "amodimethicone."

Preferably, silicone emulsion droplets are blended with certain types of surface active block polymers of a high molecular weight to form silicone emulsions, as described for example in WO03/094874. One preferred form of the surface active block polymer having polyoxypropylene and polyoxyethylene groups as the hydrophobic and hydrophilic part respectively has formula V and has the CTFA designation poloxamer, known commercially under the trade name "Pluronic" from BASF.

V) HO(CH₂CH₂O)ₓ(CH(CH₃)CH₂O)_{y}(CH₂CH₂O)ₓ H

Suitably, the mean value of x in formula I is 4 or more, preferably 8 or more, more preferably 25 or more, yet more preferably 50 or more and most preferably 80 or more. The mean value of x is typically no greater than 200. Suitably, the mean value of y is 25 or more, preferably 35 or more, more preferably 45 or more and most preferably 60 or more. The mean value of y is typically no greater than 100.

Another preferred form of the surface active block polymer is according to formula VI and has the CTFA designation Poloxamine. Those are commercially available under the trade name "Tetronic" from BASF.

VI) (HO(CH₂CH₂O)ₐ(CH(CH₃)CH₂O)_{b})₂-N-CH₂-CH₂-N-((OCH₂CH(CH₃))_{b}(OCH₂CH₂)ₐOH)₂

Suitably, the mean value of a is 2 or more, preferably 4 or more, more preferably 8 or more, even more preferably 25 or more and most preferably 40 or more. The mean value of a is typically no greater than 200. The mean value of b is suitably 6 or more, preferably 9 or more, more preferably 11 or more and most preferably 15 or more. The mean value of b is typically no greater than 50.

Preferably, the surface active block polymer is poloxamer and/or poloxamine, more preferably, the surface active block polymer is poloxamer.

Preferably, the surface active block polymer is blended with dimethicone. The weight ratio of dimethicone to surface active block polymer in the blend is preferably in the range from 2:1 to 200:1, more preferably from 5:1 to 50:1, even more preferably from 10:1 to 40:1, most preferably from 15:1 to 30:1.

The water-insoluble conditioning agent is generally present in the composition in an amount from 0.05 to 15%, preferably from 0.1 to 10%, more preferably from 0.5 to 8%, most preferably from 1 to 5%, based on the total weight of the composition and including all ranges subsumed therein.

The composition may preferably comprise a peariescer. The preferred peariescer is ethylene glycol ester as disclosed in US4885107, incorporated herein by reference. Preferably, the ethylene glycol ester is a mono- or, di-ester of glycol, more preferably a di-ester of glycol.

Preferably ethylene glycol mono- or di-ester is an ethylene glycol mono- or di-ester of a C₁₂₋₂₂ fatty acid, more preferably an ethylene glycol mono- or di-ester of a saturated C₁₂₋₂₂ fatty acid. Most preferred are the diesters comprising a mixture of palmitate and stearate. The amount of stearate should be in the range of about 10% to about 42% or in the range of about 55% to about 80% with palmitate accounting for the remainder. The amount of stearate is preferably from about 60% to about 75%, more preferably from about 80-95%, most preferably 100%. The most suitable example of a peariescer is an ethylene glycol distearate. Pearlescer may also perform the function as a suspending agent.

The level of the ethylene glycol ester can be suitably from about 0.5% to about 6%, preferably from about 1% to about 4%, by weight of the total composition.

The viscosity of the composition suitably ranges from 3,000 to 10,000 mPa.s, preferably from 4,000 to 8,000 mPa.s, more preferably from 5,000 to 7,000 mPa.s when measured using a Brookfield V2 viscometer (spindle RTV5, 1 minute, 20rpm) at 30°C.

The pH of the composition of the invention preferably ranges from 3 to 9, more preferably from 4 to 8, most preferably from 4.5 to 6.5.

Rinse-off compositions are intended to be rinsed off the scalp of the user with water after use. Rinse off compositions are preferred. Leave-on composition is intended not to be rinsed off the scalp of the user immediately after use (i.e. within at least the first 2 hours, preferably at least 4 hours after application of the composition). In the context of the present invention rinse-off compositions include shampoos and conditioners, as well as treatment compositions which can be left on scalp for from 0.5 minute to up to 15 minutes, preferably from 1 minute to 10 minutes, more preferably 1 minutes to 10 minutes, prior to being rinsed off.

The preferred use of the cosmetic composition is in a shampoo, particularly a rinse off shampoo. Shampoo compositions for use in the invention are generally aqueous, i.e. they have water or an aqueous solution or a lyotropic liquid crystalline phase as their major component. Suitably, the shampoo composition will comprise from 50 to 98%, preferably from 60 to 92% water by weight based on the total weight of the composition. Such compositions are referred to as having an aqueous base.

The invention will now be illustrated by the following non-limiting Examples.

### Examples

Compositions were made according to Table 1

**Table 1**

| **Ingredient** | **Example A Wt%** | **Example B Wt%** | **Example 1 Wt %** |
|---|---|---|---|
| sodium lauryl ether sulfate(1 EO) | 8.5 | - | - |
| sodium lauryl ether sulfate(2EO) | - | 8.5 | - |
| sodium lauryl ether sulfate(3EO) | - | - | 8.5 |
| cocamidopropyl betaine | 2.3 | 2.3 | 2.3 |
| Piroctone olamine | 0.5 | 0.5 | 0,5 |
| polyquaternium 10 | 0.2 | 0.2 | 0.2 |
| silicone DC1788 | 2 | 2 | 2 |
| Carbomer (Polyacrylic acid) | 0.4 | 0.4 | 0.4 |
| fragrance | 0.6 | 0.6 | 0.6 |
| Water and minors | To 100 | To 100 | To 100 |

The deposition of the piroctone olamine was measured by thoroughly wetting with water 10 clean, dark-brown European hair switches (2.5g/6"). 5 replicas were tested for each shampoo composition. 0.25g shampoo was applied to one switch followed by 30 second massage. The switch was then rinsed by water for 30 seconds. 0.25g shampoo was re-applied followed by another 30 second massage. The shampoo was then rinsed off by water for 30 seconds. The switch was allowed to dry naturally. The dried switch was extracted in 50ml ethanol. Caution was taken not to expose the extraction to UV light. The extraction was analysed by UPLC for piroctone acid deposition. The results are shown in Table 2.

**Table 2**

| **Example** | **Piroctone olamine deposition ppm** | **Significant difference** |
|---|---|---|
| Example A | 10.9 | No |
| Example B | 12.7 | No |
| Example 1 | 16.1 | Yes |

The results in Table 2 demonstrate that the Example of the invention (comprising an ethoxylated surfactant with a degree of ethoxylation of 3 is more effective at depositing Piroctone Olamine than the comparative Examples.

## Claims

1. A shampoo composition comprising:
i) a piroctone compound selected from piroctone acid, primary, secondary and tertiary olamine salts of piroctone acid (such as the diethanolamine and triethanolamine salts) and mixtures thereof and
ii) a cleansing surfactant comprising an amphoteric surfactant and an anionic surfactant, the anionic surfactant consisting of an ethoxylated alkyl sulphate and the total level of anionic surfactant having an average degree of ethoxylation of 3 or greater;
wherein the composition comprises a total cleansing surfactant level from 6 to 12 wt percent of the total composition; and the ratio of ethoxylated anionic surfactant to amphoteric surfactant is from 3: 1 to 4:1.

2. A shampoo composition according to claim 1 in which the anionic surfactant comprises sodium laureth sulphate.

3. A shampoo composition according to any preceding claim in which the ethoxylated alkyl sulphate is sodium laureth sulphate (3EO).

4. A shampoo composition according to any preceding claim in which the amphoteric surfactant is cocamidopropyl betaine.

5. A shampoo composition according to any preceding claim in which the piroctone compound is piroctone olamine.

6. A shampoo composition according to according to any preceding claim which further comprises a cationic polymer.

7. A shampoo composition according to any preceding claim that further comprises a silicone.

8. A shampoo composition according to any preceding claim that comprises a total level of piroctone compound from 0.1 to 5 wt percent, preferably from 0.2 to 3 wt percent, of the total composition.

9. A non-therapeutic method of treating hair or the scalp, comprising application of a shampoo composition according to any of claims 1 to 8, to the hair or scalp.

10. A non-therapeutic method according to claim 9 in which the composition is washed off after use.

11. Use of a composition as described in any one of claims 1 to 8 to enhance the deposition of a piroctone compound.

## Patentansprüche

1. Shampoozusammensetzung, umfassend:
i) eine Piroctonverbindung, ausgewählt aus Piroctonsäure, primären, sekundären und tertiären Olaminsalzen von Piroctonsäure (wie zum Beispiel Diethanolamin- und Triethanolaminsalzen) und Mischungen davon und
ii) ein Reinigungstensid, umfassend ein amphoteres Tensid und ein anionisches Tensid, wobei das anionische Tensid aus einem ethoxylierten Alkylsulfat besteht und der Gesamtanteil des anionischen Tensids einen durchschnittlichen Ethoxylierungsgrad von 3 oder mehr aufweist;
wobei die Zusammensetzung einen Gesamtanteil an Reinigungstensid von 6 bis 12 Gew.-Prozent der gesamten Zusammensetzung umfasst und das Verhältnis von anionischem ethoxyliertem Tensid zum amphoteren Tensid 3:1 bis 4:1 beträgt.

2. Shampoozusammensetzung nach Anspruch 1, in der das anionische Tensid Natriumlaurethsulfat umfasst.

3. Shampoozusammensetzung nach einem vorhergehenden Anspruch, in der das ethoxylierte Alkylsulfat Natriumlaurethsulfat (3EO) ist.

4. Shampoozusammensetzung nach einem vorhergehenden Anspruch, in der das amphotere Tensid ein Cocamidopropylbetain ist.

5. Shampoozusammensetzung nach einem vorhergehenden Anspruch, in der die Piroctonverbindung Pirocton-Olamin ist.

6. Shampoozusammensetzung nach einem vorhergehenden Anspruch, die ferner ein kationisches Polymer umfasst.

7. Shampoozusammensetzung nach einem vorhergehenden Anspruch, die ferner ein Silikon umfasst.

8. Shampoozusammensetzung nach einem vorhergehenden Anspruch, die einen Gesamtanteil an Piroctonverbindung von 0,1 bis 5 Gew.-Prozent, vorzugsweise von 0,2 bis 3 Gew.-Prozent der gesamten Zusammensetzung umfasst.

9. Nicht-therapeutisches Verfahren zur Behandlung von Haar oder der Kopfhaut, umfassend das Auftragen einer Shampoozusammensetzung nach einem der Ansprüche 1 bis 8 auf das Haar oder die Kopfhaut.

10. Nicht-therapeutisches Verfahren nach Anspruch 9, bei dem die Zusammensetzung nach dem Gebrauch abgewaschen wird.

11. Verwendung einer Zusammensetzung, wie in irgendeinem der Ansprüche 1 bis 8 beschrieben, um die Abscheidung einer Piroctonverbindung zu verstärken.

## Revendications

1. Composition de shampoing comprenant :
i) un composé de piroctone choisi parmi un acide de piroctone, des sels d'olamines primaires, secondaires et tertiaires d'acide de piroctone (tels que les sels de diéthanolamine et triéthanolamine) et mélanges de ceux-ci et
ii) un tensioactif de nettoyage comprenant un tensioactif amphotère et un tensioactif anionique, le tensioactif anionique consistant en un sulfate d'alkyle éthoxylé et la teneur totale en tensioactif anionique ayant un degré moyen d'éthoxylation de 3 ou supérieur ;
dans laquelle la composition comprend une teneur totale en tensioactif de nettoyage de 6 à 12 pourcent en masse de la composition totale ; et le rapport de tensioactif anionique éthoxylé à tensioactif amphotère est de 3:1 à 4:1.

2. Composition de shampoing selon la revendication 1, dans laquelle le tensioactif anionique comprend du laureth sulfate de sodium.

3. Composition de shampoing selon l'une quelconque des revendications précédentes, dans laquelle le sulfate d'alkyle éthoxylé est le laureth sulfate de sodium (3EO).

4. Composition de shampoing selon l'une quelconque des revendications précédentes, dans laquelle le tensioactif amphotère est la cocamidopropyl bétaïne.

5. Composition de shampoing selon l'une quelconque des revendications précédentes, dans laquelle le composé de piroctone est une piroctone olamine.

6. Composition de shampoing selon l'une quelconque des revendications précédentes qui comprend de plus un polymère cationique.

7. Composition de shampoing selon l'une quelconque des revendications précédentes qui comprend de plus un silicone.

8. Composition de shampoing selon l'une quelconque des revendications précédentes qui comprend une teneur totale en composé de piroctone de 0,1 à 5 pourcent en masse, de préférence de 0,2 à 3 pourcent en masse de la composition totale.

9. Procédé non-thérapeutique de traitement des cheveux ou du cuir chevelu, comprenant l'application d'une composition de shampoing selon l'une quelconque des revendications 1 à 8, aux cheveux ou cuir chevelu.

10. Procédé non-thérapeutique selon la revendication 9, dans lequel la composition est rincée après utilisation.

11. Utilisation d'une composition selon l'une quelconque des revendications 1 à 8 pour promouvoir le dépôt d'un composé de piroctone.
